# EUROPEAN PATENT SPECIFICATION

(11) **EP 2 483 003 B1**
(45) Date of publication and mention of the grant of the patent: **06.11.2013**
(21) Application number: 10776142.1
(22) Date of filing: 27.09.2010
(51) Int. Cl.: B09B 3/00, B09C 1/00, B03B 5/40, B03B 9/06, B03B 11/00

(54) **SYSTEM FOR PREPARING A MIXTURE OF ORGANIC FRACTION OF URBAN SOLID WASTE AND WATER**
SYSTEM FÜR DIE HERSTELLUNG EINER MISCHUNG DER ORGANISCHEN FRAKTION STÄDTISCHER FESTE ABFÄLLE UND WASSER
SYSTEME DE PREPARATION D'UN MÉLANGE DE FRACTION ORGANIQUE DE DÉCHETS SOLIDES URBAINS ET DE L'EAU

(30) Priority: 28.09.2009 IT TO20090736
(43) Date of publication of application: 08.08.2012
(73) Proprietor: ACEA Pinerolese Industriale S.p.A., 10064 Pinerolo (IT)
(72) Inventor: FARRUGGIA, Rosario, 10060 Cantalupa (IT); GENOVESIO, Sergio, 12031 Bagnolo Piemonte (IT); GENNARO, Paolo, I-10060 Frossasco (IT); MALANETTO, Alfredo, 10060 Airasca (IT); MAINERO, Davide, 10064 Pinerolo (IT); SACCO, Alberto, 10060 Airasca (IT)
(74) Representative: Civera, Andrea
(86) International application number: PCT/IB2010/002410
(87) International publication number: WO 2011/036548

(56) References cited:
- EP-A2- 1 275 443
- WO-A1-97/24186
- DE-A1- 4 312 005
- DE-A1- 19 631 250
- DE-A1-102004 024 791
- DE-U1- 9 418 405
- FR-A- 964 793

## Description

### TECHNICAL FIELD

The present invention relates to a system for preparing a mixture of organic fraction of urban solid waste (FORSU) to be fed to an anaerobic digestion process, in particular for pre-treating the organic fraction of selected urban solid waste.

### BACKGROUND ART

In an anaerobic digester, the organic fraction is degraded by microorganisms active in anaerobic conditions. As well as a solid residue which is substantially stable from a chemical point of view (digestate), this degradation generates, as a product, a gas mixture mainly containing methane and carbon dioxide, commonly referred to as biogas.

With regard thereto, this process allows the exploitation of the organic fraction of urban solid waste, since the digestate, that contains considerable amounts of nitrogen, may be used to obtain compost, while the biogas represents a source of renewable energy since it is combustible.

Following a selective waste collection of the organic fraction of urban solid waste selected as "organic" by the user, small amounts of non organic substances are generally included. For example, there are small containers and plastic bags containing food remains. Furthermore, small amounts of inert material often appear, such as cutlery, pieces of glass or pottery etc., which the user has erroneously selected for the organic fraction.

The non-biodegradable portion must be preventively separated from the organic fraction which is collected and conveyed to the treatment plant for the anaerobic digestion process, while the biodegradable portion (the specifically designated organic fraction) is generally sieved, grinded, adjusted for humidity content, reduced to a pulp, which is more or less finely subdivided, and finally fed to a digester, within which it is subjected to the action of microorganisms.

For the pre-treatment of solid waste to be fed to an anaerobic digester, systems are known in the art, e.g. from WO 97/24186 and EP 1275443.

The efficiency of the digester and, to a greater extent, the yield of the biogas, that represents the most profitable product of the entire process, are considerably influenced by the features of the organic fraction fed to the process.

The size and the geometry of the digester being the same, for example, a higher dry content in the organic fraction fed in the process corresponds to a higher production of biogas and accordingly to a greater profitability of the process.

The wet anaerobic digestion process, i.e. a process that processes an organic fraction with a dry content below 10% is generally performed in a discontinuous mode. Furthermore, since the degradation of the organic fraction in the digester progresses by subsequent steps (hydrolysis, acidogenesis and methanogenesis), in which different microbiological processes are active, and to each of which corresponds a specific composition of the gas atmosphere, the trend of the production of biogas in the course of time is generally described by a curve that has a maximum in the methanation step. Accordingly, from the point of view of profitability of the process, the more the production of biogas oscillates, the more discontinuous is the anaerobic digestion process.

The need is therefore felt in the field to homogenise the organic substance to be fed to the digester as far as composition and size are concerned, in order to manage the biological degradation process in a continuous-like manner. As a matter of fact, a more homogeneous and substantially continuous feeding would result in a greater uniformity in the yield of biogas of the process. The final result is a greater profitability and ease in the management of the plant.

The need is also felt in the field to pre-treat the organic fraction of urban solid waste from the selective waste collection so that a mixture having an increased dry content is obtained and can be fed to the digester. This would result in the increase of the yield of biogas of the anaerobic digestion process. The need is also felt for a pre-treated organic fraction from which the residual non biodegradable fractions have been removed with high efficiency, without having to use complicated systems that imply several separation stages.

Furthermore, the need is felt in the field to reduce the size of the apparatuses used to perform the pre-treatment operations of the organic fraction of urban solid waste and to perform the anaerobic digestion process, in order to better exploit space and to reduce costs.

### DISCLOSURE OF INVENTION

It is an object of the present invention to therefore provide a system for pre-treating solid waste to be fed to an anaerobic digester, which system allows to satisfy in a simple and cost-effective manner at least one of the above said needs.

The above said object is achieved by the present invention, as it relates to a system according to claim 1.

### BRIEF DESCRIPTION OF THE DRAWINGS

Some preferred embodiments are hereinafter disclosed for a better understanding of the present invention, by mere way of non-limitative example and with reference to the accompanying drawings, in which:
- Figure 1 is a diagrammatic section view which shows a system for preparing the mixture of organic fraction and water to be fed to an anaerobic digestion process according to the invention;
- Figure 2 is a diagrammatic section view which shows an alternative system for preparing the mixture of organic fraction and water to be fed to an anaerobic digestion process which is not part of the invention; and
- Figure 3 is a diagrammatic section view that shows a variant of the system of Figure 2.

### - BEST MODE FOR CARRYING OUT THE INVENTION

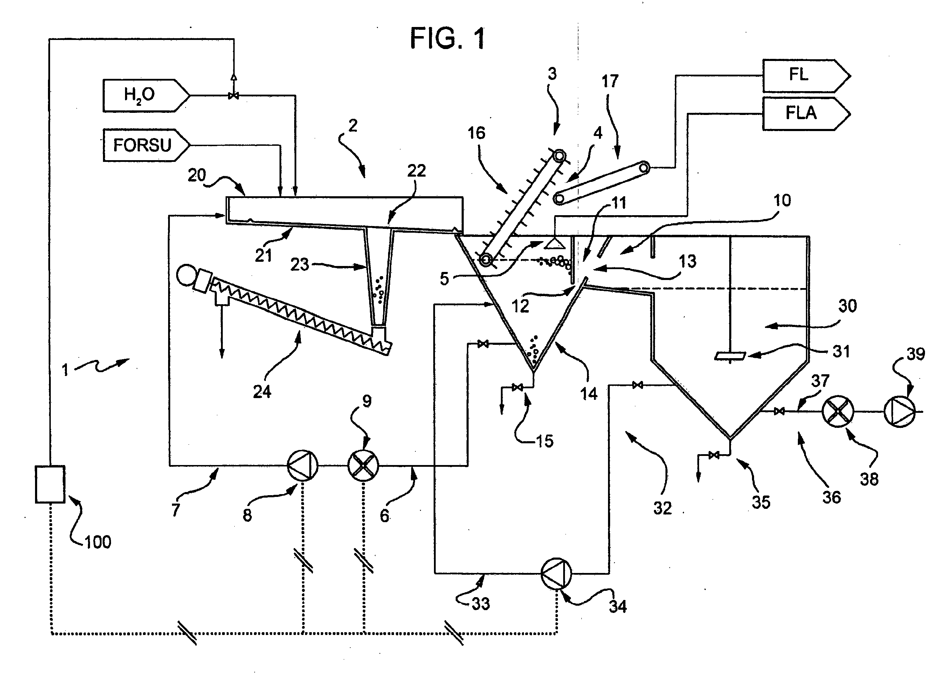

With reference to figure 1, numeral 1 indicates as a whole a system for preparing a mixture of solid wastes from a selective waste collection and water to be fed to an anaerobic digestion process.

System 1 comprises a first separation stage 2 for separating the heavy fraction of the organic fraction of urban solid waste, in particular a fraction comprising materials having a density higher than water.

Advantageously, first stage 2 comprises a conveying channel 20 for conveying the mixture of organic fraction of urban solid waste and water to be pre-treated. Channel 20 has a bottom wall 21 inclined downwards in the feeding direction of the mixture of organic fraction of urban solid waste and water. The degree of inclination of bottom wall 21 is selected so as to ensure that the mixture of organic fraction of urban solid waste and water moves forward by gravity along the channel at a speed compatible with the sedimentation of the heavy fraction contained in the mixture.

Furthermore, first stage 2 comprises a trap 22 for capturing this heavy fraction formed for example by a channel 23 directed along a substantially vertical axis and having an opening having a size compatible with the passage of items included in the above said heavy fraction. First stage 2 finally comprises means 24 to remove the heavy fraction captured in channel 23, for example consisting of a screw conveyor arranged with the inlet mouth at the outlet of channel 23 which is distal with respect to the bottom wall of conveying channel 20.

System 1 further comprises a second separation stage 3 for separating the light fraction of the organic fraction of urban solid waste, in particular a fraction comprising materials having a density lower than water (supernatant).

More specifically, second stage 3 comprises first separation means 4 configured to remove a light fraction of the organic fraction of urban solid waste contained in the mixture which at least partially emerges from the free surface in second stage 3. Generally, this fraction comprises plastic materials which were not separated during the selective waste collection and have been fed together with the organic fraction. For example, this portion comprises small containers and plastic bags used as containers for food products, etc. First separation means 4 may comprise a conveyor belt having the lower end arranged in the portion of second stage 3 proximal to the outlet of first stage 2, so as to effectively collect the floating plastic materials immediately after their entry in second stage 3.

Furthermore, first separation means 4 can comprise suction means 5 by means of which small floating materials may be removed even when they considerably emerge above the free surface.

Therefore, a first light fraction, indicated in Figure 1 by FL, mechanically removed by a comb and/or belt conveyor, and a second light fraction, indicated in Figure 1 by FLA, sucked by suction means 5 collect at the outlet of second stage 3.

Advantageously, system 1 may further comprise means 6 for recirculating a portion of the mixture from second stage 3 to first stage 2. These recirculation means 6 generally comprise a hydraulic line 7, along which are provided a pump 8 for conveying fluid and a grinder 9 in which the average size of the solid part suspended in the mixture is reduced and equalized, in order to handle the same in an easier way and aid the following steps of treating, in particular the actual digestion step. Advantageously, grinder 9 is arranged upstream of pump 8.

Second stage 3 further comprises second separation means 10 for the organic fraction of urban solid waste present in the mixture. These second means 10 comprise an outlet duct 11 of the mixture from second stage 3, duct 11 having a first mouth 12 arranged, in use (in a steady state), below the free surface of a decantation tank 14 in second stage 3, and a second mouth 13 arranged at a level between the free surface in the second stage and first mouth 12.

Advantageously, the decantation tank 14 of second stage 3 has a conical bottom, adapted to receive the flow of mixture from the first stage at an upper edge of its side wall. The decantation tank is fluidically connected to first stage 2 by first recirculation means 6 which have been disclosed above. Furthermore, decantation tank 14 is provided with drainage means 15 of the known type, located at a lower portion thereof. A comb conveyor 16 is mounted above decantation tank 14 with a lower end thereof positioned within tank 14 so as to collect the supernatant fraction. Comb conveyor 16 is configured to remove the supernatant fraction and transfer it downstream for its recovery, for example by means of a belt conveyor 17.

Decantation tank 14 further comprises a wall 18 lying on a plane substantially parallel to the axis of the conical portion of tank 14 and not comprising this axis. Advantageously, first mouth 12 of outlet duct 11 is defined between a lower edge of wall 18 and the conical wall of tank 14. Advantageously, second mouth 13 of outlet duct 11 is made in the form of an overfall obtained in the conical side wall of tank 14.

Outlet duct 11 is advantageously in hydraulic communication with a homogenisation chamber 30 comprising stirring means 31 for the mixture of organic fraction of urban solid waste and treated water, from which the light and heavy fractions are separated in the first and second stage 2 and 3.

Preferably, homogenisation chamber 20 comprises a conical bottom portion.

Advantageously, system 1 comprises means 32 for recirculating a portion of the treated mixture from homogenisation chamber 30 to second stage 3. These recirculation means 32 generally comprise a hydraulic line 33 along which a pump 34 is arranged for conveying fluid.

Furthermore, homogenising tank 30 is provided with drainage means 35 of the known type, located at a lower vertex of the conical portion thereof.

Finally, homogenisation tank 30 is provided with means 36 for ejecting the treated mixture from chamber 30 and for then feeding the same to the process of anaerobic digestion. In general, these means 36 may comprise a hydraulic line 37, along which a grinder 38 and a pump 39 are provided in series.

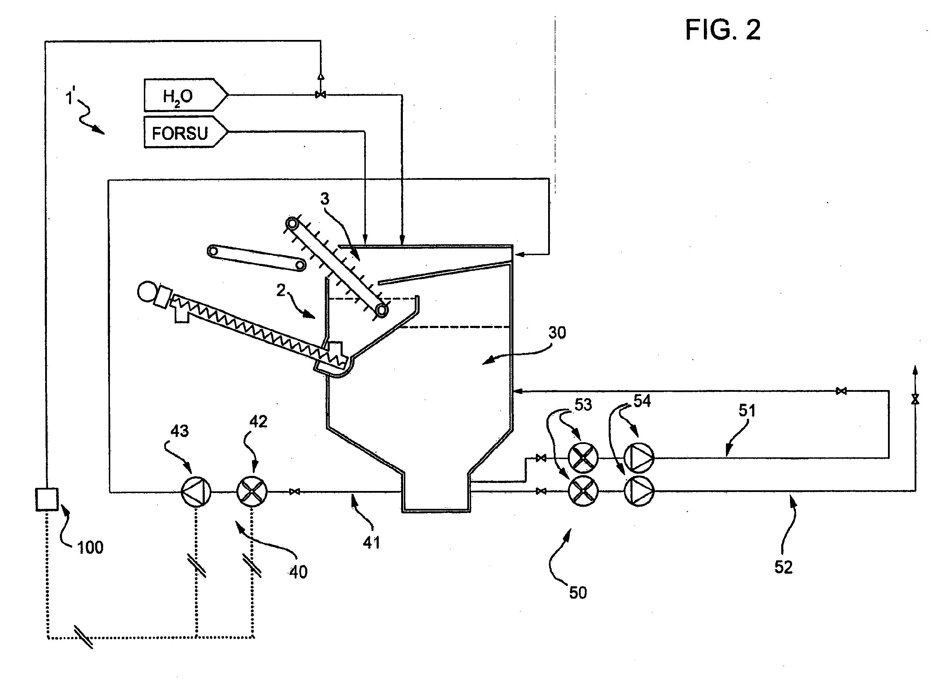

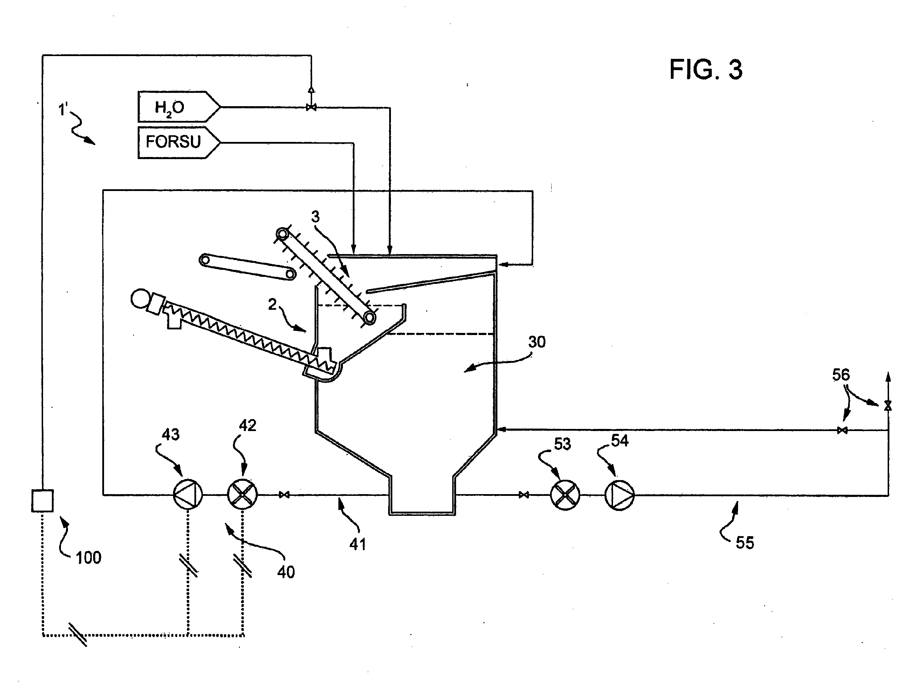

Figures 2 and 3 show two variants of an alternative system 1' which is not part of the invention. In this alternative system, first separation stage 2 and second separation stage 3 are obtained in a homogenising tank 30, thus obtaining a reduction of the volume taken up. System 1' comprises means 40 for recirculating a fraction of the mixture treated upstream of first separation stage 2. These mans 40 comprise a hydraulic line 41, along which a grinder 42 and a pump 43 are arranged.

Furthermore, system 1' comprises discharge and/or recirculation means 50. According to a first variant, shown in Figure 2, these means 50 comprise two distinct hydraulic lines 51 and 52, respectively intended for the recirculation and discharge. Respective grinders 53 and pumps 54 are arranged in series on each of the lines.

According to the variant shown in Figure 3, discharge and/or recirculation means 50 comprise a single hydraulic line 55, along which are arranged a grinder 53 and a pump 54 and valve means 56 for the selective conveying of a flow of treated mixture to the anaerobic digestion process downstream and/or backwards, to the homogenisation chamber 30.

The energy absorbed by the recirculation means of system 1 (pumps and grinders) is directly influenced by density, viscosity and composition (in particular by the dry amount) of the flow of mixture that is materially recirculated. With regard thereto, the measurement of the energy absorbed by pumps and grinders may be considered an indirect measure of the dry amount of the mixture of organic fraction of urban solid waste. This information may advantageously be used to adjust the flow of water entering system 1 (replenishment water). For this purpose, system 1 may comprise a control system 100 configured to adjust the entering water flow rate as a function of the measurement of the energy absorbed by pumps and grinders.

Advantageously, system 1 further comprises a pneumatic circuit (not shown) of the known type and adapted to selectively convey a controlled flow of network air (at a pressure on the order of 5÷7 bar) to first separation stage 2, to second separation stage 3 and to homogenisation chamber 30, in order to facilitate the suspension of the content of the various elements of system 1. Preferably, this controlled air flow is introduced through corresponding inlets obtained in respective lower portions of first and second stage 2 and 3 and of chamber 30. For example, this air flow is introduced in first separation stage 2 at a lower portion of trap 22; at a lower portion of decantation tank 14; and at a lower conical portion of the conical portion of homogenisation tank.

In use, a flow of mixture of organic fraction of urban solid waste and water to be pre-treated is conveyed to first stage 2, where it is fed along inclined channel 20. At trap 22, heavy items (fragments, pieces of glass, etc.) are captured and removed by means of screw conveyor 24. The mixture, at this point containing no heavy fraction, is fed towards second separation stage 3, where the light fraction emerging above the free surface is removed from comb conveyor 16, while light particles having a finer granulometry are removed by suction means 5.

The geometry of tank 14 is such as to promote the sedimentation on the bottom of heavy particles having fine granulometry which may not be separated by trap 22 at the previous stage. Furthermore, the geometry of second stage 3 and, in particular, of outlet duct 11 is such as to retain light objects (plastic objects and the like) within second stage 3, avoiding that the same proceed downstream to homogenisation tank 30.

The mixture of organic fraction of urban solid waste and treated water collects in homogenisation chamber 30, where it is stirred. Furthermore, ventilation means may be provided in each of the stages of the system. These ventilation means have the dual purpose of stirring the mixture and promoting the floating of light particles towards the free surface.

From an analysis of the characteristics of the system according to the present invention, the advantages it allows to obtain are apparent.

In particular, the system of the invention allows to feed to the anaerobic digestion process a mixture of water and biodegradable fraction of the organic fraction of urban solid waste, from which the non-biodegradable parts incorrectly disposed of upon selective waste collection have been efficiently removed. Furthermore, the special structural and functional configuration of second stage 3 allows to increase the amount of dry in the treated mixture up to values of 12-14%, which result, in the following step of anaerobic digestion, in an increase of the yield of biogas and in the profitability of the process, the volumes of the treated mixture and the volume of the apparatuses being the same.

Furthermore, the system of the invention allows to obtain a better degree of homogeneity of the pre-treated mixture, both in terms of composition, as the light and heavy non biodegradable fractions are substantially removed, and in terms of suspended particle size, as the possibility of repeatedly grinding the mixture is provided when the mixture passes from a pre-treatment stage to the other. When the flow rates of the organic fraction of urban solid waste and water to the pre-treatment system and the corresponding outflow of pre-treated mixture are appropriately adjusted, this allows to feed a process of anaerobic digestion continuously, ensuring that the system has a homogenous condition compatible with such an operation mode of the digester. Accordingly, the system of the invention allows a trend of the productivity of biogas which is less oscillating, therefore promoting the profitability of the process of anaerobic digestion.

Since the separation of the heavy and light fraction in first and second separation stages 2 and 3 of the system of the invention is especially efficient, the amount of recirculated material that needs to be grinded is reduced with respect to the known solutions. With regard thereto, the system of the invention allows a reduction of the energy consumption for switching on the grinders. Furthermore, since the fluidity of the recirculated mixture is accordingly higher, the load on the pumps and therefore the energy consumption for switching the pumps on is also accordingly reduced.

Finally, it is clear that modifications and variants not departing from the scope of protection of the claims can be made to the disclosed and shown system.

## Claims

1. A system for preparing a mixture of organic fraction of urban solid waste and water, the mixture being for feeding an anaerobic digestion process, said system comprising a first separation stage (2) for separating a first heavy fraction of said urban solid waste and a second separation stage (3), **characterised in that** said second separation stage (3) comprises first (4,5) and second (10) separation means for said urban solid waste, said first means (4,5) being configured to remove a light fraction of said urban solid waste, the light fraction at least partially emerging from the free surface of the mixture of organic fraction of urban solid waste and water in a decantation tank (14) of the second stage, said second means (10) comprising an outlet duct (11) for said mixture from said second stage (3); the outlet duct (11) having a first mouth (12) arranged below the free surface of the mixture of organic fraction of urban solid waste and water in the decantation tank (14) of the second stage (3) and a second mouth (13) arranged at a level between the free surface of the mixture of organic fraction of urban solid waste and water in the decantation tank (14) of the second stage (3) and said first mouth (12).

2. The system according to claim 1, **characterised in that** said outlet duct (11) is in hydraulic communication with a homogenising chamber (30) comprising stirring means (31) for said mixture.

3. The system according to claim 1 or 2, **characterised in that** said first stage (2) comprises means (22, 23) for separating by gravity said heavy fraction from said mixture and for discharging (24) said separate heavy fraction.

4. The system according to any of claims 1 to 3, **characterised in that** said first separation means (4) for separating the light fraction comprise at least one of a comb conveyor (16) and a suction system (5).

5. The system according to any of claims 1 to 4, **characterised by** comprising means (6) for recirculating a portion of said mixture from said second stage (3) to said first stage (2).

6. The system according to one of claims 1 to 5, **characterised by** comprising means (32) for recirculating a portion of said mixture from said homogenising chamber (30) to said second stage (3).

7. The system according to one of claims 5 or 6, **characterised by** comprising a control system (100) configured to adjust a water flow rate entering the system (1) as a function of an energy absorption signal by said means (6, 32) for recirculating said mixture.

## Patentansprüche

1. Ein System zur Herstellung einer Mischung aus einem organischen Anteil festen städtischen Abfalls und Wasser, wobei die Mischung zur Einspeisung in einen anaerobischen Abbauprozess vorgesehen ist, und wobei das System aufweist: eine erste Trennstufe (2) zum Trennen eines ersten schweren Anteils des erwähnten festen städtischen Abfalls und eine zweite Stufe (3) aufweist, **dadurch gekennzeichnet, dass** die erwähnte zweite Trennstufe (3) erste (4, 5) und zweite (10) Trennmittel für den festen städtischen Abfall aufweist, wobei die ersten Mittel (4, 5) konfiguriert sind, um einen leichten Anteil des festen städtischen Abfalls zu entfernen, wobei der leichte Anteil zumindest teilweise von der freien Oberfläche der Mischung des organischen Anteils festen städtischen Mülls und Wasser in einem Dekantierungstank (14) der zweiten Stufe austritt, wobei die erwähnten zweiten Mittel (10) eine Auslassleitung (11) für die erwähnte Mischung von der zweiten Stufe (3) aufweisen; wobei die Auslassleitung (11) eine erste Mundöffnung (12) aufweist angeordnet unterhalb der freien Oberfläche der Mischung aus dem organischen Anteil des festen städtischen Abfalls und Wasser in dem Dekantierungstank (14) der zweite Stufe (3) und eine zweite Mundöffnung (13) angeordnet auf einem Niveau zwischen der freien Oberfläche der Mischung aus dem organischen Anteil des festen städtischen Abfalls und Wasser in dem Dekantierungstank (14) der zweiten Stufe (3) und der ersten Mundöffnung (12).

2. System nach Anspruch 1 **dadurch gekennzeichnet, dass** die Auslassleitung (11) in hydraulischer Verbindung mit einer Homogenisierungskammer (30) steht, die Rührmittel (31) für die erwähnte Mischung aufweist.

3. Das System nach Anspruch 1 oder 2, **dadurch gekennzeichnet, dass** die erwähnte erste Stufe (2) Mittel (22, 23) aufweist zum Trennen durch Schwerkraft des erwähnten schweren Anteils von der Mischung und zur Abgabe (24) des erwähnten getrennten schweren Anteils.

4. Das System nach einem der Ansprüche 1 bis 3, **dadurch gekennzeichnet, dass** die erwähnten ersten Trennmittel (4) zur Trennung des leichten Anteils mindestens eine Kammtransportvorrichtung (16) und ein Saugsystem (15) aufweisen.

5. Das System nach einem der Ansprüche 1 bis 4, **dadurch gekennzeichnet, dass** Mittel (6) vorgesehen sind zum Rezirkulieren eines Teils der erwähnten Mischung aus der erwähnten zweiten Stufe (3) zur erwähnten ersten Stufe (2).

6. Das System nach einem der Ansprüche 1 bis 5 **dadurch gekennzeichnet, dass** Mittel (32) vorgesehen sind zur Rezirkulierung eines Teils der erwähnten Mischung von der erwähnten Homogenisierungskammer (30) zu der erwähnten zweiten Stufe (3).

7. Das System nach einem der Ansprüche 5 oder 6 **dadurch gekennzeichnet, dass** ein Steuersystem (100) vorgesehen ist, konfiguriert zur Einstellung einer in das System (1) eintretenden Wasserflussrate als eine Funktion eines Energie-Absorptionssignals durch die Mittel (6, 32) zur Rezirkulation der Mischung

## Revendications

1. Système de préparation d'un mélange d'une fraction organique de déchets urbains solides et d'eau, le mélange étant destiné à alimenter un procédé de digestion anaérobie, ledit système comprenant un premier étage de séparation (2) pour séparer une première fraction lourde desdits déchets urbains solides et un second étage de séparation (3), **caractérisé en ce que** ledit second étage de séparation (3) comprend des premiers (4, 5) et seconds (10) moyens de séparation pour lesdits déchets urbains solides, lesdits premiers moyens (4, 5) étant configurés pour éliminer une fraction légère desdits déchets urbains solides, la fraction légère émergeant au moins partiellement de la surface libre du mélange de fraction organique de déchets urbains solides et d'eau dans une cuve de décantation (4) du second étage, lesdits seconds moyens (10) comprenant un conduit de refoulement (11) pour ledit mélange depuis ledit second étage (3) ; le conduit de refoulement (11) ayant une première embouchure (12) agencée en dessous de la surface libre du mélange de fraction organique de déchets urbains solides et d'eau dans la cuve de décantation (14) du second étage (3) et une seconde embouchure (13) agencée à un niveau entre la surface libre du mélange de fraction organique de déchets urbains solides et d'eau dans la cuve de décantation (14) du second étage (3) et ladite première embouchure (12).

2. Système selon la revendication 1, **caractérisé en ce que** ledit conduit de refoulement (11) est en communication hydraulique avec une chambre d'homogénéisation (30) comprenant des moyens d'agitation (31) pour ledit mélange.

3. Système selon la revendication 1 ou 2, **caractérisé en ce que** ledit premier étage (2) comprend des moyens (22, 23) de séparation par gravité de ladite fraction lourde dudit mélange et d'évacuation (24) de ladite fraction lourde séparée.

4. Système selon l'une quelconque des revendications 1 à 3, **caractérisé en ce que** lesdits premiers moyens de séparation (4) pour séparer la fraction légère comprennent au moins l'un d'un convoyeur à peigne (16) et d'un système d'aspiration (5).

5. Système selon l'une quelconque des revendications 1 à 4, **caractérisé en ce qu'**il comprend des moyens (6) pour faire recirculer une portion dudit mélange depuis ledit second étage (3) vers ledit premier étage (2).

6. Système selon l'une des revendications 1 à 5, **caractérisé en ce qu'**il comprend des moyens (32) pour faire recirculer une portion dudit mélange depuis ladite chambre d'homogénéisation (30) vers ledit second étage (3).

7. Système selon l'une des revendications 5 ou 6, **caractérisé en ce qu'**il comprend un système de régulation (100) configuré pour régler un débit d'eau entrant dans le système (1) en fonction d'un signal d'absorption d'énergie par lesdits moyens (6, 32) pour faire recirculer ledit mélange.
